# EUROPÄISCHE PATENTANMELDUNG

(11) **EP 1 407 813 A1**
(43) Veröffentlichungstag der Anmeldung: **14.04.2004**
(21) Anmeldenummer: 03022145.1
(22) Anmeldetag: 27.09.2003
(51) Int. Cl.: B01F 17/42, B01F 17/00, A61K 7/46

(54) **Polyethylenglykol enthaltende homogene Mikroemulsion**

(30) Priorität: 07.10.2002 DE 10246706
(71) Anmelder: Clariant GmbH, 65929 Frankfurt am Main (DE)
(72) Erfinder: Miller, Dennis, Dr., 65779 Kelkheim (DE); Henning, Torsten, Dr., 65812 Bad Soden (DE)
(74) Vertreter: Paczkowski, Marcus, Dr.

(57) **Zusammenfassung**

Eine homogene, durchscheinende Mikroemulsion enthält
a) 1 bis 70 Gew.-% einer wasserunlöslichen Flüssigkeit;
b) 1 bis 98 Gew.-% eines Polyethylenglykols;
c) 0 bis 97 Gew.-% Wasser; und
d) 1 bis 20 Gew.-% eines Tensids

Die Mikroemulsion ist thermodynamisch stabil und eignet sich bevorzugt zum Herstellen von kosmetischen Mitteln, bevorzugt von Badeöl, oder von Reinigungsmitteln.

## Beschreibung

### Homogene Mikroemulsion enthaltend Polyethylenglykol

Die vorliegende Erfindung betrifft eine homogene, durchscheinende Mikroemulsion, die Polyethylenglykol enthält.

Das Phasenverhalten von Kohlenwasserstoff/Tensid/Wasser-Systemen ist Gegenstand zahlloser Untersuchungen. Durch geeignete Auswahl der einzelnen Komponenten lassen sich Mikroemulsionen erhalten, bei denen größere Mengen an Kohlenwasserstoff im wässrigen Medium solubilisiert sind. Derartige Mikroemulsionen sind durchscheinend, womit die Begriffe transparent oder transluzent synonym zusammengefasst sind und werden beispielsweise beschrieben in
- H.G. Hauthal und K. Quitsch "Neues über Mikroemulsionen", Z. Chem., 30, 274 - 281 (1990).

Ähnliche Phänomene wie bei den Mikroemulsionen werden auch bei einigen anderen nicht-polaren Flüssigkeiten beobachtet. In der Literatur wird die nicht-polare Flüssigkeit allgemein mit dem Begriff "Öl" oder "Ölphase" umschrieben.

In der Praxis ist die Formulierung einer Mikroemulsion oft schwierig. Für die Bildung der Mikroemulsion muss das Tensid für das jeweilige System optimiert werden. Das geschieht z.B., indem man die Länge der Polyethylenoxidkette eines Oxethylats verändert. Weiter muss eine ausreichende Menge an Tensid vorhanden sein, um das Öl zu solubilisieren. Je größer die Ölmoleküle sind, umso schwieriger ist es, eine Mikroemulsion zu formulieren. Je nach Zusammensetzung kann die Zugabe eines kurzkettigen Amphiphils (Cotensid) wie z.B. Butanol notwendig sein. Schließlich sind manche Formulierungen nur über einen beschränkten Temperaturbereich stabil.

Überraschend wurde gefunden, dass durch den vollständigen oder teilweisen Ersatz des Wassers durch Polyethylenglykol (PEG) die Bildung einer Mikroemulsion ermöglicht oder zumindest erleichtert werden kann.

Die Erfindung betrifft daher eine Mikroemulsion der eingangs genannten Gattung, deren Kennzeichenmerkmal darin zu sehen ist, dass sie folgende Bestandteile enthält:
a) 1 bis 70 Gew.-%, bevorzugt 10 bis 50 Gew.-% einer wasserunlöslichen Flüssigkeit;
b) 1 bis 98 Gew.-%, bevorzugt 20 bis 80 Gew.-%, eines Polyethylenglykols;
c) 0 bis 97 Gew.-%, bevorzugt 0 bis 60 Gew.-%, Wasser; und
d) 1 bis 20 Gew.-%, bevorzugt 2 bis 15 Gew.-%, eines Tensids mit der allgemeinen chemischen Formel:

   R-(OCHR'CH)ₙOH,

   in der R für einen Alky- oder Alkenylrest mit 8 bis 22 C-Atomen steht, vorzugsweise mit 12 bis 18 C-Atomen, oder für einen Alkylphenol- oder Polyalkylphenolrest mit 4 bis 16 Alkyl-C-Atomen,
   in der R' für H oder CH₃ oder eine Mischung davon steht, vorzugsweise für H,
   und in der n = eine ganze Zahl im Bereich von 2 bis 20 ist,
   wobei alle Angaben in Gew.-% bezogen sind auf das Gesamtgewicht der Mikroemulsion.

In der erfindungsgemäßen Mikroemulsion wird die Mindestmenge an Tensid, Komponente d), soweit wie möglich reduziert, zusätzlich ist die Zugabe eines Cotensids, eines kurzkettigen Alkohols, nicht nötig. Der Vorteil des Einsatzes von PEG kann auch darin bestehen, dass die Mikroemulsion über einen breiteren HLB-Bereich (= Hydrophilic/Lipophilic Balance Value) des Tensids gebildet wird, wodurch eine wesentlich größere Freiheit bei der Ausarbeitung der Formulierung entsteht.

Polyethylenglykole, die für die erfindungsgemäße Mikroemulsion als Komponente b) geeignet sind, besitzen bevorzugt eine mittlere Molmasse im Bereich von 150 bis 35 000 g/mol, bevorzugt von 200 bis 800 g/mol, gemessen durch Bestimmung der OH-Zahlen von 600 bis 1 mg KOH/g, Vorzugsbereich: 591 bis 134 mg KOH/g. Bei den Tensiden, die für die erfindungsgemäße Mikroemulsion als Komponente d) geeignet sind, kann es sich um nicht-ionische, kationische, anionische und/oder amphotere Tenside handeln.

Bei den nicht-ionischen Tensiden handelt es sich bevorzugt um Fettalkoholethoxylate, Dimethylaminoxide, ethoxylierte Rizinusöle, Alkylpolyglucoside, Fettsäuresorbitolester, Fettsäurepolyglycerolester, ethoxylierte Fettsäurepolyglycerolester, Fettsäuremonoethanolamidethoxylate, Glycerinmonound -diestern von Fettsäuren und/oder Phosphorsäuretriester.

Ebenfalls bevorzugt als nicht-ionische Tenside sind (C₈-C₂₂)-Alkyl- oder Alkenylethoxylate mit 2 bis 20 Ethylenoxidgruppen.

Bei den anionischen Tensiden handelt es sich bevorzugt um Phosphorsäuremonoester, Phosphorsäurediester, Alkylsulfate, Alkylethersulfate, bevorzugt Natriumlaurethsulfat, Alkylamidopolyglykolethersulfate, Alkylpolyglykolethercarboxyate, Alkylpolyglykolethersulphosuccinate und/oder Fettsäureisethionate.

Bei den amphoteren Tensiden handelt es sich bevorzugt um Acylglutamate, Alkylamidopropylbetaine, bevorzugt Cocoamidopropylbetain, Fettsäuremethyltauride, Fettsäuresarcoside und/oder Amphoacetate.

In einer besonderen Ausführungsform handelt es sich bei den Tensiden um Betaine, Alkylethersulfate oder Mischungen aus diesen.

Als wasserunlösliche Flüssigkeit, die für die erfindungsgemäße Mikroemulsion als Komponente a) geeignet ist, eignen sich bevorzugt Mineralöle, Polydecene, Triglyceride, z.B. Capric/Caprylic Triglyceride, natürliche Öle, z.B. Orangenöl, und/oder Ester, bevorzugt Stearate, Palmitate und Myristate.

Die wasserunlösliche Flüssigkeit der Komponente a) ist bevorzugt in der erfindungsgemäßen Mikroemulsion mit einem Solubilisierungsgrad S von größer oder gleich 0,8, bevorzugt größer oder gleich 1,5, solubilisiert. Bei dem Solubilisierungsgrad S handelt es sich um das Volumenverhältnis der Komponente a) zu dem Tensid der Komponente d).

In einer besonderen Ausführungsform kann die Mikroemulsion noch zusätzlich polare organische Verbindungen, bevorzugt Hydroxyverbindungen und/oder Polyhydroxyverbindungen, besonders bevorzugt Glycerin, Propylenglykol, Ethanol, Hexylenglykol und/oder Isopropanol in jeweils geeigneten Mengen enthalten.

In einer weiteren besonderen Ausführungsform kann die Mikroemulsion noch wasserlösliche und/oder öllösliche, bevorzugt verschiedenfarbige, Farbstoffe enthalten. Hierdurch lassen sich vorteilhafte optische Effekte ästhetischer Art erzielen.

Die erfindungsgemäßen Mikroemulsionen eignen sich besonders für Kosmetik- und Haushaltsformulierungen. Ein besonderer Vorteil dabei ist, dass Polyethylenglykol im Gegensatz zu manchen anderen Cotensiden weder toxisch noch feuergefährlich ist.

Im Bereich der Kosmetik haben die Mikroemulsionen folgende Anwendungen:
- Badeöle oder Duschgele mit rückfettender Wirkung;
- Spezielle Hautpflegemittel;
- Solubilisierung von Wirkstoffen, z.B. in Sonnenschutzmitteln.

Kosmetische Formulierungen können auch je nach Anwendungszweck noch weitere Bestandteile, wie z.B. pflanzliche Extrakte, Vitamine, Antioxidantien, Hydroxycarbonsäuren, Fettalkohole, Allantoin, Verdicker oder Konsistenzgeber, organische oder anorganische UV-Absorber, Parfüm, Konservierungsstoffe, Farbstoffe, Perlglanzmittel, kationische Polymere, Organosilikone, (z.B. Caprylyl Trimethicone, Phenyltrimethicone) und pH-Regulatoren enthalten.

Die erfindungsgemäßen Mikroemulsionen sind auch als Reinigungsmittel geeignet, z.B. für besonders hartnäckigen Schmutz an harten Oberflächen, oder für die Vorbehandlung von Flecken vor der Wäsche von Kleidungsstücken.

Reinigungsmittel enthalten, je nach Anwendungszweck, weitere Bestandteile, wie z.B. Natriumcitrat, Zeolithe, anorganische Phosphate, Komplexiermittel, Abrasivstoffe, Polyacrylsäuren und deren Salze, Farbschutzadditive, Säuren bzw. Laugen, Triethanolamin, Elektrolyte, optische Aufheller, Verdicker, Parfüm, Farbstoffe, Konservierungsmittel, Lösemittel, Enzyme, Desinfektionsmittel und Entschäumer.

Weitere Anwendungen der erfindungsgemäßen Mikroemulsionen sind:
- Solubilisierung von Wirkstoffen in pharmazeutischen und agrochemischen Formulierungen;
- Spezielle Lösungsmedien für die präparative und analytische Chemie.

Bei manchen technischen Anwendungen möchte man mit möglichst wenig Tensid möglichst viel Öl in die Mikroemulsion einbringen. Das maximale Volumenverhältnis Öl zu Tensid, das definiert ist als Solubilisierungsgrad S des Öls, kann man als Maß für die Wirksamkeit des Tensids nehmen. S hängt unter anderem von der chemischen Zusammensetzung des Öls ab. Diese Betrachtungsweise ist aber nur dann angebracht, wenn das Volumen des Öls geringer ist als das Volumen der polaren Flüssigkeit (hier PEG-Wasser-Gemisch).

Das Herstellen der erfindungsgemäßen Mikroemulsion kann vorteilhaft durch einfaches Mischen der einzelnen Komponenten a), b), ggf. c) und d) erfolgen. Das thermodynamische Gleichgewicht der Mikroemulsion stellt sich nach dem Mischen von selbst ein.

In einer bevorzugten Ausführungsform werden die hydrophilen Komponenten (Polyethylenglykole, Wasser, wasserlösliche Farbstoffe etc.), einschließlich der Tenside, miteinander vermischt. Separat davon werden die hydrophoben, wasserunlöslichen Komponenten (Öle, öllösliche Farbstoffe etc.) miteinander vermischt. Anschließend werden die hydrophile Mischung und die hydrophobe Mischung miteinander vermischt.

### Ausführungsbeispiele

Beispiele 1 und 2 zeigen erfindungsgemäße Mikroemulsionen.

"Oleth-5", "Oleth-8" und "Oleth-10" sind die INCI-Bezeichnungen (INCI = International Nomenklatura of Cosmetic Ingredients) für Oleylalkoholpolyglykolether mit 5 oder 8 oder 10 Mol Ethylenoxid.
PEG-8 ist die INCI-Bezeichnung für Polyethylenglykol mit mittlerer Molmasse von 400. Alle Mengenangaben sind in Gew.-%.

**Tabelle 1**

| Beispiel 1 | | Beispiel 2 | |
|---|---|---|---|
| Komponente | Gew.-% | Komponente | Gew.-% |
| Paraffinöl | 38,6 | Dekan | 36,9 |
| Oleth-5 | 6,5 | Oleth-8 | 10,1 |
| Oleth-8 | 6,5 | Oleth-10 | 1,4 |
| PEG-8 | 19,4 | PEG-8 | 20,6 |
| Wasser | 29,0 | Wasser | 30,9 |
| Aussehen | Homogene transparente Flüssigkeit | Aussehen | Homogene transparente Flüssigkeit |

Die Zusammensetzungen der vorstehend aufgeführten Beispiele 1 und 2 wurden in Volumenanteile umgerechnet, damit man die Effektivität des Tensids genauer beurteilen kann. Das Ergebnis ist nachfolgend in Tabelle 2 dargestellt.

**Tabelle 2**

| Komponente | Beispiel 1 | Beispiel 2 |
|---|---|---|
| | Vol % | Vol % |
| Öl | 43,7 | 44,7 |
| Polare Flüssigkeit (PEG/Wasser) | 43,7 | 44,7 |
| Tensid | 12,6 | 10,6 |

Im Falle von Beispiel 2 wurde in zusätzlichen Versuchen der EO-Grad (= Ethoxylierungsgrad) variiert, indem das Verhältnis von Oleth-8 : Oleth-10 bzw. Oleth-5 : Oleth-8 geändert wurde. Das Phasenverhalten wurde auch bei verschiedenen Temperaturen untersucht. Dabei hat sich gezeigt, dass das Phasenverhalten, das ein Anzeichen für die Stabilität der Mikroemulsion ist, weitgehend unabhängig ist von der Temperatur.

### Beispiel 3

Dieses Beispiel zeigt jeweils Vergleichsmessungen mit und ohne PEG. Es wurde geprüft, ob es einen optimalen Bereich des EO Grades gibt, bei der eine Mikroemulsion gebildet wird. Um den EO Grad zu variieren, wurden entsprechend Mischungen von Tensiden mit verschieden EO Graden benutzt (Oleth-2, Oleth-5, Oleth-8 und Oleth-10).
Erfindungsgemäße Mikroemulsion (Prozentangaben in Gew.-%, bezogen auf Gesamtgewicht):

| | |
|---|---|
| Paraffinöl | 16,0 % |
| Capric Caprylic Triglyceride | 4,0 % |
| Oleth-5 | 4,0 % |
| Oleth-8 | 4,0 % |
| PEG-8 | 28,8 % |
| Wasser | 43,2 % |

Die Mischung bildete eine homogene transparente Flüssigkeit.

### Vergleichsversuch:

In einem Vergleichsversuch wurde das PEG-8 durch Wasser ersetzt. Der mittlere EO Grad des Tensids wurde von 2 bis 10 variiert. Es wurde keine Bildung von Mikroemulsionen beobachtet.

## Patentansprüche

1. Homogene, durchscheinende Mikroemulsion, die Polyethylenglykol enthält, **dadurch gekennzeichnet, dass** sie:
a) 1 bis 70 Gew.-%, bevorzugt 10 bis 50 Gew.-% einer wasserunlöslichen Flüssigkeit enthält;
b) 1 bis 98 Gew.-%, bevorzugt 20 bis 80 Gew.-%, eines Polyethylenglykols;
c) 0 bis 97 Gew.-%, bevorzugt 0 bis 60 Gew.-%, Wasser; und
d) 1 bis 20 Gew.-%, bevorzugt 2 bis 15 Gew.-%, eines Tensids mit der allgemeinen chemischen Formel:
R-(OCHR'CH)ₙOH,
in der R für einen Alkyl- oder Alkenylrest mit 8 bis 22 C-Atomen steht, vorzugsweise mit 12 bis 18 C-Atomen, oder für einen Alkylphenol- oder Polyalkylphenolrest mit 4 bis 16 Alkyl-C-Atomen,
in der R' für H oder CH₃ oder eine Mischung davon steht, vorzugsweise für H,
und in der n eine ganze Zahl im Bereich von 2 bis 20 bedeutet,
wobei alle Angaben in Gew.-% bezogen sind auf das Gesamtgewicht der Mikroemulsion.

2. Mikroemulsion nach Anspruch 1, **dadurch gekennzeichnet, dass** das Gewichtsverhältnis Polyethylenglykol : Wasser im Bereich von 99 : 1 bis 30 : 70 liegt.

3. Mikroemulsion nach Anspruch 1 oder 2, **dadurch gekennzeichnet, dass** die wasserunlösliche Flüssigkeit weniger als 50 Vol.-% der Mikroemulsion beträgt und das Volumenverhältnis wasserunlösliche Flüssigkeit zu Tensid mindestens 2,0 beträgt.

4. Mikroemulsion nach einem oder nach mehreren der Ansprüche 1 bis 3, **dadurch gekennzeichnet, dass** der Polyethylenglykol eine Molmasse im Bereich von 150 bis 35 000 g/mol, bevorzugt von 200 bis 800 g/mol, besitzt.

5. Mikroemulsion nach einem oder nach mehreren der Ansprüche 1 bis 4, **dadurch gekennzeichnet, dass** sie als Komponente a), die wasserunlösliche Flüssigkeit, Öle, Kohlenwasserstoffe, bevorzugt Mineralöl oder Polydecene, Triglyceride, bevorzugt natürliche Öle und/oder Ester, bevorzugt Stearate, Palmitate und Myristate enthält.

6. Mikroemulsion nach einem oder nach mehreren der Ansprüche 1 bis 5, **dadurch gekennzeichnet, dass** sie als Komponente d) ein Tensid ausgewählt aus nicht-ionischen, kationischen, anionischen und amphoteren Tensiden enthält.

7. Mikroemulsion nach Anspruch 6, **dadurch gekennzeichnet, dass** sie als nichtionischenes Tensid Dimethylaminoxide, ethoxylierte Rizinusöle, Poloxamer, Alkylpolyglucoside, Fettsäuresorbitolester, Fettsäurepolyglycerolester, ethoxylierte Fettsäurepolyglycerolester, Fettaminethoxylate, Fettsäuremonoethanolamidethoxylate, Glycerinmono- und -diestern von Fettsäuren und/oder Phosphorsäuretriester enthält.

8. Mikroemulsion nach Anspruch 6, **dadurch gekennzeichnet, dass** sie als anionisches Tensid Phosphorsäuremonoester, Phosphorsäurediester, Alkylsulfate, Alkylbenzolsulfonate, Alkansulfonate, Alkylpolyglykolethersulfate, bevorzugt Natriumlaurethsulfat, Alkylamidopolyglykolethersulfate, Alkylpolyglykolethercarboxyate, Alkylpolyglykolethersulphosuccinate und/oder Fettsäureisethionate enthält.

9. Mikroemulsion nach Anspruch 6, **dadurch gekennzeichnet, dass** sie als amphoteres Tensid Acylglutamate, Alkylamidopropylbetaine, bevorzugt Cocoamidopropylbetain, Fettsäuremethyltauride, Fettsäuresarcoside und/oder Amphoacetate enthält.

10. Mikroemulsion nach einem oder nach mehreren der Ansprüche 1 bis 9, **dadurch gekennzeichnet, dass** die Komponente a) in der Mikroemulsion mit einem Solubilisierungsgrad S von größer oder gleich 0,8, bevorzugt größer oder gleich 1,5, solubilisiert ist.

11. Mikroemulsion nach einem oder nach mehreren der Ansprüche 1 bis 10, **dadurch gekennzeichnet, dass** sie zusätzlich einen Elektrolyt enthält.

12. Mikroemulsion nach einem oder nach mehreren der Ansprüche 1 bis 11, **dadurch gekennzeichnet, dass** sie zusätzlich polare organische Verbindungen, bevorzugt Hydroxyverbindungen und/oder Polyhydroxyverbindungen, besonders bevorzugt Glycerin, Propylenglykol, Ethanol und/oder Isopropanol enthält.

13. Verwendung einer Mikroemulsion nach einem oder nach mehreren der Ansprüche 1 bis 12 für kosmetische Formulierungen.

14. Verwendung einer Mikroemulsion nach einem oder nach mehreren der Ansprüche 1 bis 12 für Reinigungsmittel.
